# EUROPEAN PATENT SPECIFICATION

(11) **EP 1 455 687 B1**
(45) Date of publication and mention of the grant of the patent: **10.09.2014**
(21) Application number: 02792459.6
(22) Date of filing: 18.12.2002
(51) Int. Cl.: A61F 2/07, A61F 2/00, A61F 2/848, A61F 2/89

(54) **STENT GRAFT WITH IMPROVED GRAFT ADHESION**
STENT-GRAFT MIT VERBESSERTER GRAFTHAFTUNG
ENDOPROTHESE VASCULAIRE A ADHERENCE AMELIOREE

(30) Priority: 19.12.2001 AU PR961701
(43) Date of publication of application: 15.09.2004
(73) Proprietor: Cook Medical Technologies LLC, Bloomington, IN 47404 (US)
(72) Inventor: HARTLEY, David, Ernest, Subiaco, Western Australia 6008 (AU)
(74) Representative: Jehan, Robert
(86) International application number: PCT/US2002/040663
(87) International publication number: WO 2003/053287

(56) References cited:
- WO-A-02/22055
- WO-A1-94/01056
- WO-A2-02/35988
- US-A- 4 871 366
- US-A- 6 152 956
- US-A1- 2002 052 649

## Description

### Technical Field

This invention relates to endoluminal stent grafts and in particular to a stent graft for improving the adhesion or incorporation of such grafts into the wall of a lumen.

### Background of the Invention

Throughout this specification when applied to a blood vessel the term distal, with respect to a stent graft or graft, is the end of the stent graft or graft furthest away in the direction of blood flow from the heart within a body lumen. The term proximal means the end of the stent graft or graft nearest to the heart in the direction of blood flow. Where the invention is applied to other lumens of the human or animal body then corresponding terms such as caudal and cranial should be understood.

A surgical stent graft or graft may be placed into a lumen in the body by endoluminal techniques or by surgical techniques. Such a stent graft or graft is constructed from biocompatible materials but it is desirable in the long term for such grafts to be actually incorporated into the wall of the body lumen and this requires that the tissue of the body lumen grows into the material of the graft.

It is known that tufts of fibrous material may be placed on grafts to enhance blood clotting and adhesion but such tufts do not promote complete circumferential adhesion. Particularly when such grafts are deployed endoluminally it is desirable that the amount of fibrous material be kept to a minimum to ensure that the graft can be compressed into small enough volume to enable deployment while at the same time ensuring good adhesion of the graft.

US 6,152,956 discloses an apparatus for endovascular repair comprising an anchoring unit for placement at a location away from an aneurysm, connected by thin lines to a tube to serve as a conduit in the aneurysm. In one embodiment, a collar is provided to the tube to seal any small spaces between the tube and the neck of the aneurysm.

### Summary of the Invention

The object of this invention is to provide a graft with material to ensure good adhesion or incorporation of the graft into a lumen.

According to an aspect of the invention, there is provided a stent graft as in claim 1.

The band of fibrous material extends circumferentially around the graft to promote adhesion and incorporation. Furthermore, the graft or stent graft can include separate bands of fibrous material extending circumferentially around the graft at both the distal and the proximal ends thereof to promote adhesion and incorporation.

Preferably the band is continuous around the proximal end of the graft.

The band of fibrous material is provided by fibres woven or knitted into the material of the graft.

Embodiments of the invention include a stent graft adapted for insertion into an internal lumen of a patient to be incorporated therein, the stent graft comprising a substantially tubular body providing a fluid flow path and having a periphery defined by a biocompatible relatively impervious material wall, the wall having a proximal portion including a continuous external circumferential band of a fibrous biocompatible material.

Embodiments of the invention include a stent graft adapted for implantation within a lumen in a human or animal body to reside therein, the stent graft having a proximal end and a distal end and a substantially tubular graft body formed from a relatively impervious biocompatible material, at least one stent to maintain the patency of the stent graft, and a continuous circumferential band of fibrous material on the outside of the tubular graft body adjacent the proximal end of the stent graft.

It would be seen that by the various forms of the invention there is provided fibrous material around the outside of the proximal end of a stent graft for good adhesion of that end of the stent graft to the wall of a lumen.

It will be noted that the graft of the present invention is particularly useful in delivery devices when endoluminal deployment is used because the band is of a relatively short length with respect to the length of the graft and hence does not make the device when it is compressed for deployment significantly larger over the entire length of the device.

The band of fibrous material is fibres woven or knitted into the material of the graft.

To ensure good adhesion the band may be in the region of 5 mm to 15 mm in length along the length of the graft.

In one preferred form of the invention, the invention is particularly related to a stent graft using self-expanding stents such as stainless steel or nitinol stents.

The relatively impervious material may be a knitted or woven biocompatible material such as a dacron or similar material or it may be a non-woven fibrous material or it may be a plastics material sheet formed into a graft tube or extruded into tubular form.

It is desirable that the band be continuous around the proximal end of the graft so that with fibrous ingrowth of the cells of the wall of the lumen into the band of fibrous material on the graft, union of the wall and graft is obtained which provides sealing for blood flow which could occur between the graft and the wall as well as prevention of movement of the graft longitudinally within the lumen. In so doing there is improved incorporation of the graft into the wall of the lumen.

### Brief Description of the Drawing

This then generally describes the invention but to assist with understanding reference will now be made to the accompanying drawing which show preferred embodiments of the invention.

In the drawings,
Figure 1 shows a perspective view of a stent graft including the adhesion or incorporation arrangement according to one embodiment of the present invention;
Figure 2 shows a perspective view of a stent graft including an adhesion or incorporation arrangement;
Figure 3A shows a plan view of a stent graft shown in Figure 1 with a first form of velour pile;
Figure 3B shows a plan view of a stent graft shown in Figure 1 with an alternative form of velour pile; and
Figure 4 shows a detailed perspective view of a stent graft including an adhesion or incorporation arrangement.

### Detailed Description

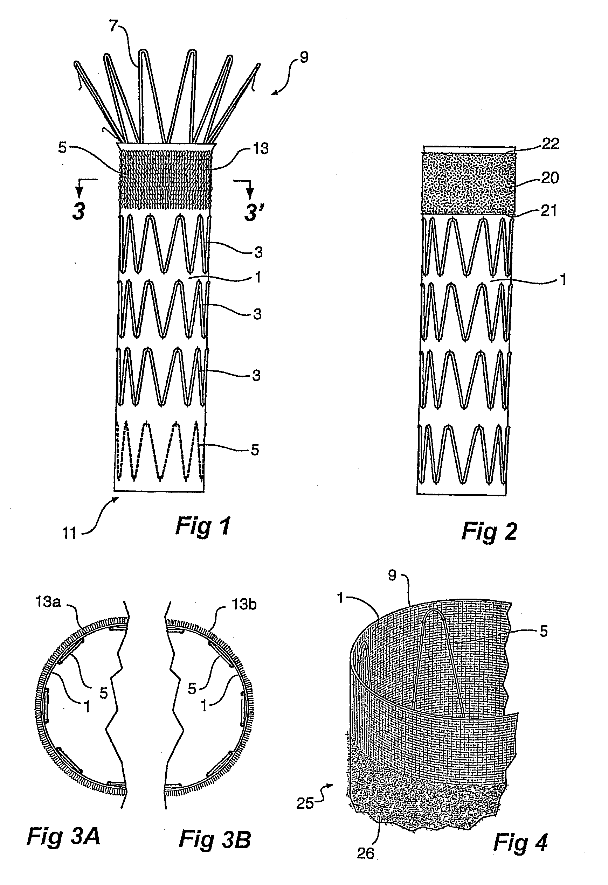

Looking more closely at Figure 1 of the drawings it can be seen that in a stent graft comprising a graft material tube 1 which is held into a substantially cylindrical shape by means of external expandable stents 3 in the main body of the graft material 1 and internal stents 5 at each end of the graft. These stents can be self-expanding of, for example, commercially available Gianturco™, zigzag or Z stent using a spring like metal such as stainless steel, nitinol, or other alloy metals. These stents can also be balloon or mechanically expandable stents, which are also commercially available.

The graft body has a proximal end 9 and a distal end 11.

In this particular embodiment, a proximally extending stent 7 is provided although this proximally extending stent is not essential to the invention.

Each stent 3, 5 and 7 is a Gianturco™ zig zag or Z stent manufactured from nitinol or stainless steel and is self expanding from a compressed state, in which the stent graft can be introduced into the lumen, to the expanded state shown.

Extending circumferentially around the graft body 1 at the proximal end 9 is a continuous circumferential region 13 of fibres extending from the graft material. The continuous circumferential region is formed on the material of the graft 1 by portions of the fibrous material woven into the material or extending from the woven graft material. The portions of fibrous material may be in the form of a loop pile or a cut pile to present either loops of fibres or individual fibres to the wall of the vessel when deployed within a body lumen.

The graft may have a diameter in the range of 25 to 50 millimetres and a length of from 100 to 200 millimetres. The band 13 may have a length of from 5 to 15 millimetres and may be adjacent the end of the stent graft or spaced from the proximal end of the graft by a distance of up to 10 millimetres.

Figure 2 shows an example useful for explaining the invention. In this example the graft 1 is of substantially the same construction as shown in Figure 1 except that it does not have a proximally extending uncovered stent. Around the proximal end 19 of the graft 1 is a continuous band of velour material 20 fastened to the graft 1 by stitching 22 at the proximal end and 21 at the distal end of the velour band 20. The velour provides loops of fibrous material which can be incorporated into the wall of the lumen into which the graft is placed by fibrous ingrowth. This provides adhesion of the graft into the lumen. One again the velour material may be of loop pile or cut pile form.

The provision of the continuous band at the proximal end of the graft provides adhesion and incorporation around the entire circumference of the graft. The adhesion and incorporation around the entire circumference of the graft will prevent blood or other body fluid, depending upon the lumen into which it is introduced, from getting behind the wall of the graft. The adhesion and incorporation will also assist with preventing movement of the graft within the lumen.
Figure 3A and B show cross sectional plan views in the line 3-3'on the stent graft shown in Figure 1 but with different forms of velour pile.
Figure 3A shows loop pile velour as the continuous band 13a of fibrous material around the circumference of the graft 1.
Figure 3B shows cut pile velour as the continuous band 13b of fibrous material around the circumference of the graft 1.
Figure 4 shows a detailed perspective view of part of a stent graft including an alternative adhesion arrangement for the proximal end of the stent graft. As with Figure 1 the stent graft has a bio-compatible woven material body 1 with a self expanding stent 5 inside the graft body 1 at the proximal end 9 to provide a good contact of the graft material with the wall of a lumen into which it is deployed. In this case the continuous band of fibrous material 25 is provided by brushing or similar means to raise fibres 26 from the material of the graft 1. The brushing can be with a wire brush or a hooking system may be used to raise some of the fibres in a continuous band around the graft.

This brushing or hooking can also be used with non-woven bio-compatible graft materials to raise a fibrous band around the circumference of a graft.

It may be noted that this specification has been particularly directed to the use of a velour band on the proximal end of a graft because that is the end of the graft which it is normally expected to get more blood pressure in the case where the lumen is a blood vessel and the proximal end in the case of an artery being the end nearer the heart. There may be situations, however, where the band of fibrous material may be placed around the distal end of a graft or at both ends and this invention is intended to cover those applications as well.

Throughout this specification various indications have been given as to the scope of this invention but the invention is not limited to any one of these but may reside in two or more of these combined together. The examples are given for illustration only and not for limitation.

Throughout this specification and the claims that follow unless the context requires otherwise, the words 'comprise' and 'include' and variations such as 'comprising' and 'including' will be understood to imply the inclusion of a stated integer or group of integers but not the exclusion of any other integer or group of integers.

## Claims

1. A stent graft comprising:
a tubular graft (1) formed from a relatively impervious biocompatible material having a distal end (11), a proximal end (9), and an outer surface;
at least one expandable stent (3) disposed on said tubular graft (1); and
a band of fibrous material (13) on said outer surface and adjacent at least one of said distal end and said proximal end of said tubular graft (1), said band of fibrous material extending circumferentially around said graft; wherein said band of fibrous material is provided by fibres woven or knitted into material of said graft, said band of fibrous material being arranged to promote ingrowth of tissue into the graft.

2. The stent graft as in claim 1, wherein said band is continuous around said proximal end of said tubular graft.

3. The stent graft as in claim 1 or 2, wherein each stent is a nitinol or stainless steel stent.

4. The stent graft as in claim 1 wherein said band of fibrous material has a length of from 5mm to 15 mm along the length of said graft.

5. The stent graft as in claim 1 wherein said at least one expandable stent (7) extends proximally from said proximal end (9) of said tubular graft.

6. The stent graft as in claim 1 wherein said stent graft further comprises another band of fibrous material on said outer surface and adjacent a remaining one of said distal end and said proximal end of said tubular graft, said other band of fibrous material extending circumferentially around said graft.

## Patentansprüche

1. Stentgraft, umfassend:
einen röhrenförmigen Graft (1), der aus einem relativ undurchlässigen biokompatiblen Material mit einem distalen Ende (11), einem proximalen Ende (9) und einer Außenfläche gebildet ist,
mindestens einen expandierbaren Stent (3), der auf dem röhrenförmigen Graft (1) angeordnet ist, und
ein Band aus fasrigem Material (13) auf der Außenfläche und neben dem distalen Ende und/oder dem proximalen Ende des röhrenförmigen Grafts (1), wobei sich das Band aus fasrigem Material umfangsmäßig um den Graft herum erstreckt, wobei das Band aus fasrigem Material durch Fasern bereitgestellt ist, die in das Material des Grafts hineingewebt oder -gewirkt sind, wobei das Band aus fasrigem Material dazu angeordnet ist, das Hineinwachsen von Gewebe in den Graft zu fördern.

2. Stentgraft nach Anspruch 1, wobei das Band um das proximale Ende des röhrenförmigen Grafts herum durchgehend ist.

3. Stentgraft nach Anspruch 1 oder 2, wobei jeder Stent ein Nitinol- oder Edelstahlstent ist.

4. Stentgraft nach Anspruch 1, wobei das Band aus fasrigem Material eine Länge von 5 mm bis 15 mm entlang der Länge des Grafts hat.

5. Stentgraft nach Anspruch 1, wobei sich der mindestens eine expandierbare Stent (7) proximal vom proximalen Ende (9) des röhrenförmigen Grafts erstreckt.

6. Stentgraft nach Anspruch 1, wobei der Stentgraft ferner ein weiteres Band aus fasrigem Material auf der Außenfläche und neben einem verbleibenden des distalen Endes und des proximalen Endes des röhrenförmigen Grafts umfasst, wobei sich das weitere Band aus fasrigem Material umfangsmäßig um den Graft herum erstreckt.

## Revendications

1. Endoprothèse vasculaire comprenant :
une greffe tubulaire (1) formée d'un matériau biocompatible relativement imperméable ayant une extrémité distale (11), une extrémité proximale (9), et une surface extérieure ;
au moins un stent extensible (3) disposé sur ladite greffe tubulaire (1) ; et
une bande de matériau fibreux (13) sur ladite surface extérieure et adjacente à au moins l'une de ladite extrémité distale et de ladite extrémité proximale de ladite greffe tubulaire (1), ladite bande de matériau fibreux s'étendant sur la circonférence autour de ladite greffe ; ladite bande de matériau fibreux étant pourvue de fibres tissées ou tricotées dans le matériau de ladite greffe, ladite bande de matériau fibreux étant prévue pour favoriser la croissance des tissus à l'intérieur de la greffe.

2. Endoprothèse vasculaire selon la revendication 1, dans laquelle ladite bande est continue autour de ladite extrémité proximale de ladite greffe tubulaire.

3. Endoprothèse vasculaire selon la revendication 1 ou 2, dans laquelle chaque stent est un stent en nitinol ou en acier inoxydable.

4. Endoprothèse vasculaire selon la revendication 1, dans laquelle ladite bande de matériau fibreux présente une longueur de 5 mm à 15 mm le long de la longueur de ladite greffe.

5. Endoprothèse vasculaire selon la revendication 1, dans laquelle ledit au moins un stent extensible (7) s'étend proximalement depuis ladite extrémité proximale (9) de ladite greffe tubulaire.

6. Endoprothèse vasculaire selon la revendication 1, dans laquelle ladite endoprothèse vasculaire comprend en outre une autre bande de matériau fibreux sur ladite surface extérieure et adjacente à l'une restante de ladite extrémité distale et de ladite extrémité proximale de ladite greffe tubulaire, ladite autre bande de matériau fibreux s'étendant circonférentiellement autour de ladite greffe.
